(19) Europäisches Patentamt
European Patent Office
Office européen des brevets

(11) Veröffentlichungsnummer: **0 147 848 B1**

(12) **EUROPÄISCHE PATENTSCHRIFT**

(45) Veröffentlichungstag der Patentschrift: **13.11.91**

(51) Int. Cl.5: **G01N 33/543**, G01N 33/563, G01N 33/577

(21) Anmeldenummer: **84116335.5**

(22) Anmeldetag: **27.12.84**

Die Akte enthält technische Angaben, die nach dem Eingang der Anmeldung eingereicht wurden und die nicht in dieser Patentschrift enthalten sind.

(54) **Verfahren zur Bestimmung eines polyvalenten Antigens und Reagenz hierfür.**

(30) Priorität: **02.01.84 DE 3400027**
**06.07.84 DE 3425008**

(43) Veröffentlichungstag der Anmeldung:
**10.07.85 Patentblatt 85/28**

(45) Bekanntmachung des Hinweises auf die Patenterteilung:
**13.11.91 Patentblatt 91/46**

(84) Benannte Vertragsstaaten:
**AT BE CH DE FR GB IT LI LU NL SE**

(56) Entgegenhaltungen:
**EP-A- 0 052 769**
**EP-A- 0 098 590**
**EP-A- 0 105 714**
**US-A- 4 292 403**

**CLINICAL CHEMISTRY, Band 27, Nr. 6, 1981, Seiten 823-827; M. USATEGUI-GOMEZ et al.: "Immunochemical determination of CK-MB isoenzyme in human serum: A radiometric approach"**

(73) Patentinhaber: **BOEHRINGER MANNHEIM GMBH**
**Patentabteilung, Abt. E Sandhofer Strasse 112-132 Postfach 31 01 20**
**W-6800 Mannheim 31 Waldhof(DE)**

(72) Erfinder: **Baier, Manfred**
**Tiefentalweg 10**
**W-8124 Seeshaupt(DE)**
Erfinder: **Klose, Sigmar**
**Breitenloh 7**
**W-8131 Berg 2(DE)**
Erfinder: **Schlumberger, Helmut**
**Kaiser Heinrich-Str. 23**
**W-8121 Polling(DE)**
Erfinder: **Kleemann, Wolfgang**
**Alpspitzstr. 7**
**W-8132 Tutzing(DE)**
Erfinder: **Pasch, Manfred**
**Anton Bartl-Str. 7**
**W-8132 Tutzing(DE)**
Erfinder: **Vieth, Friedhelm**
**Hauptstr. 11**
**W-8121 Haunshofen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

(74) Vertreter: **Huber, Bernhard, Dipl.-Chem. et al Patentanwälte H. Weickmann, Dr. K. Fincke F.A. Weickmann, B. Huber Dr. H. Liska, Dr. J. Prechtel Möhlstrasse 22 Postfach 860 820 W-8000 München 86(DE)**

**Beschreibung**

Die Erfindung betrifft ein immunchemisches Verfahren zur Bestimmung eines polyvalenten, d. h. mindestens bifunktionellen Liganden (Antigen) in einer flüssigen Probe.

Die empfindliche Bestimmung von polyvalenten Antigenen (Peptide, Proteine) unter Verwendung von zwei Antikörpern, die gegen unterschiedliche Antigendeterminanten gerichtet sind, ist bekannt als 2-Site immunoradiometischer bzw. immunoenzymometrischer Assay z. B. aus J. Clin. Chem. Clin. Biochem. 18 - (1980) 197-208. Am gebräuchlichsten ist die Durchführung dieses bekannten Bestimmungsverfahrens, indem zuerst das zu bestimmende Antigen mit einem ersten Antikörper, der in fester Phase vorliegt durch Bindung an ein geeignetes Trägermaterial wie Sepharose, Agarose, Kunststoffröhrchen oder dergleichen, inkubiert wird. Während dieser ersten Inkubation bindet der erste Antikörper, der in der Regel in großem Überschuß vorhanden sein muß, mit einer Antigendeterminante des zu bestimmenden Antigens. Dann wird üblicherweise die Probeflüssigkeit von der festen Phase getrennt, um Störungen durch nicht spezifische Substanzen wie Humanserumprotein bzw. durch kreuzreagierende Antigene in der anschließenden zweiten Inkubation auszuschalten. Dann wird eine bestimmte Menge eines zweiten markierten Antikörpers in flüssiger Phase mit der festen Phase inkubiert. Dabei wird die Spezifität des zweiten Antikörpers bevorzugt so gewählt, daß sie gegen eine andere Determinante des zu bestimmenden Antigens gerichtet ist als der erste Antikörper, um eine Konkurrenz zwischen den beiden Antikörpern um die selben Bindungsstellen an dem zu bestimmenden Antigen auszuschließen, da hierdurch die Testempfindlichkeit beeinträchtigt würde.

Während dieser zweiten Inkubation reagiert der markierte zweite Antikörper, der ebenfalls regelmäßig im Überschuß vorliegt, mit sämtlichen Bindungsstellen an Molekülen des zu bestimmenden Antigens. Nach der zweiten Inkubation kann die Aktivität der Markierungssubstanz entweder an der festen Phase oder im Überstand gemessen werden. Üblicherweise erfolgt die Messung dabei an der festen Phase nach Auswaschung der flüssigen Phase. Im günstigsten Fall verhält sich hierbei die bestimmte Aktivität fast proportional zur Menge des zu bestimmenden Antigens.

Dieses 2-Schritt-Sandwich-Verfahren hat den Vorteil, daß eventuell kreuzreagierende Antigene bereits bei der ersten Phasentrennung entfernt werden. Dies ist von besonderer Bedeutung bei Testsystemen, bei denen die beiden Antikörper wegen der Empfindlichkeitsanforderungen wirklich unterschiedliche Spezifität besitzen müssen. In diesem Fall besteht die hohe Anforderung an die Spezifität nur für einen der beiden Antikörper, der andere muß dann nicht absolut spezifisch gegenüber kreuzreagierenden Antigenen sein.

Dieses Verfahren hat jedoch drei wesentliche Nachteile.

1. Im ersten Inkubationsschritt liegt ein Reaktant in fester Phase vor, der andere in Lösung. Die Geschwindigkeitskonstante der Reaktion ist damit geringer als wenn sich beide Reaktionsteilnehmer (Antigen und erster Antikörper) in Lösung befänden. Es muß jedoch so lange inkubiert werden, bis alle Antigenmoleküle an die feste Phase gebunden vorliegen, da nicht gebundenes Antigen bei der Phasentrennung entfernt würde und damit eine Verfälschung des Resultats zur Folge hätte.

2. Die Bindung eines für das Antigen spezifisch bindefähigen Antikörpers an eine Festphase erfordert wegen der geringen Bindungsausbeuten relativ große Mengen an erstem Antikörper, der meist eine sehr kostbare Substanz ist, insbesondere wenn das Antiserum aus Spezifitätsgründen in Kleintieren, wie Kaninchen, Meerschweinchen und dergleichen, gewonnen werden muß. Außerdem können bei der Bindungsreaktion an die Festphase Affinitätsverschlechterungen am Antikörper entstehen, was sich nachteilig auf die Empfindlichkeit auswirkt.

3. Die Gewinnung von zwei Antikörpern unterschiedlicher Spezifität gegen das gleiche Antigen ist in der Regel aufwendig und vielfach nur begrenzt möglich, beispielsweise indem man zwei verschiedene Tierspezies immunisiert oder indem man die Antikörper-Populationen aus einem Tier durch Immunadsorption an dem zu bestimmenden Antigen in geeignete Fragmente aufzutrennen versucht.

Es sind bereits verschiedene Versuche bekannt, die obigen Mängel zu beheben. So wird gemäß US-PS 40 98 876 die erste Inkubation des Antigens mit Isotopen-markiertem gelöstem Antikörper durchgeführt. Erst danach wird der Festphasen-gebundene Antikörper hinzugefügt und die zweite Inkubation vorgenommen. Hier ist nur noch eine Phasentrennung erforderlich, was eine gewisse Erhöhung der Empfindlichkeit und Praktikabilität ergibt. Das Spezifitätsproblem bleibt jedoch ungelöst, da beide Antikörper hochspezifisch gegen das Antigen gerichtet sein müssen, da sonst in Anwesenheit größerer Mengen kreuzreagierender Antigene die scheinbare Konzentration des zu bestimmenden Antigens bei einer etwaigen Unspezifität des zweiten Antikörpers erhöht wird, bei Unspezifität des ersten Antikörpers herabgesetzt.

In der DE-OS 29 25 565 wird ein Verfahren beschrieben, bei dem der in fester Phase vorliegende erste Antikörper und der markierte gelöste zweite Antikörper gleichzeitig mit dem Antigen inkubiert werden. Hier bestehen die gleichen Probleme wie bei dem zuvor genannten Verfahren. Außerdem bleiben auch die oben für das Basisverfahren beschriebenen Nachteile 2 und 3 ungelöst.

Aus der offengelegten japanischen Patentanmeldung 123 802 vom 06.10.1978 ist schließlich ein Verfahren bekannt, bei welchem ein Festphasen-Antikörper verwendet wird, der spezifisch gegen den zweiten Antikörper gerichtet ist, welcher mit dem zu messenden Antigen bindefähig ist. Hier wird die Bindung eines gegen das Antigen gerichteten Antikörpers an die feste Phase vermieden. Jedoch wird die Empfindlichkeit herabgesetzt, da nur ein einziger mit dem Antigen spezifisch bindefähiger Antikörper verwendet wird. Der Konzentrationsnachweis des zu bestimmenden Antigens erfolgt über die kompetitive Reaktion zwischen dem Antigen und einer bestimmten Menge an Isotopen-markiertem Antigen mit dem spezifischen Antikörper.

Aus der EP-A 105 714 ist ein Verfahren bekannt, bei welchem ein Festphasenantikörper zusammen mit zwei weiteren Antikörpern, von denen mindestens einer ein monoklonaler Antikörper sein muß, nach dem Sandwich-Prinzip zur Antigenbestimmung verwendet wird. Dabei wird das Antigen zuerst mit den beiden in flüssiger Phase vorliegenden Antikörpern und danach mit dem Festphasen-Antikörper inkubiert. Das Verfahren hat den Nachteil, zwei Inkubationsschritte zu erfordern, was der Automatisierung hinderlich ist, zu langen Inkubationszeiten zu führen und die Verwendung unverdünnter Proben nicht zuzulassen. Ein ähnliches Verfahren ist aus Clin. Chem. 27/6, (1981) 823-827 bekannt. Dort wird eine radiometrische Bestimmungsmethode für Creatinkinase MB-Isoenzym beschrieben, bei welcher in einem ersten Inkubationsschritt das Antigen gleichzeitig mit einem unmarkierten und mit einem markierten Antikörper unterschiedlicher Spezifität und aus unterschiedlichen Tierspezies umgesetzt wird. Im zweiten Schritt wird dann ein in fester Phase vorliegender Antikörper mit Spezifität gegen den unmarkierten Antikörper des ersten Inkubationsschrittes zugesetzt. Der Nachteil dieses Verfahrens besteht wiederum darin, daß zwei verschiedene für das Antigen spezifische Antikörper und drei verschiedene Tierspezies benötigt werden.

Der Erfindung liegt daher die Aufgabe zugrunde, einen oder mehrere der oben geschilderten Nachteile des Basisverfahrens besser zu vermeiden als die bisher bekannten Verfahren. Insbesondere soll ein derartiges Verfahren geschaffen werden, welches nur eine Inkubation erfordert, keinen der Antikörper in flüssiger Phase einsetzen muß, sich für die Automatisierung eignet und hohe Empfindlichkeit und Genauigkeit aufweist. Weiter soll es das Verfahren ermöglichen, gegebenenfalls mit einer einzigen Tierspezies für die Gewinnung der erforderlichen Antikörper auszukommen bzw. für die Antiserumgewinnung Großtiere verwenden zu können.

Diese Aufgabe wird erfindungsgemäß gelöst durch ein Verfahren zur Bestimmung eines polyvalenten Antigens durch Inkubation mit drei verschiedenen Rezeptoren, von denen der erste und der dritte in flüssiger Phase gelöst vorliegen und mit dem Antigen bindefähig sind, der zweite Rezeptor in fester Phase vorliegt und nur mit einem Teil des ersten Rezeptors bindefähig ist und der dritte Rezeptor eine Markierung trägt und mit dem ersten und zweiten Rezeptor nicht kreuzreagiert, Trennung der festen von der flüssigen Phase und Messung der Markierung in einer der Phasen, das dadurch gekennzeichnet ist, daß man eine das Antigen enthaltende Lösung mit einem ersten festen Trägermaterial zusammenbringt, welches den ersten und den dritten Rezeptor in ablösbarer Form imprägniert oder lyophilisiert enthält und nach Ablösung der Rezeptoren die erhaltene Lösung sofort mit einem zweiten festen Trägermaterial kontaktiert, welches den zweiten Rezeptor in nicht ablösbarer Form enthält, danach die Flüssigkeit vom zweiten festen Trägermaterial abtrennt und die Markierung mißt, wobei man als dritten Rezeptor entweder ein Fab-Antikörperfragment verwendet, das aus der gleichen Tierspezies wie der Antikörper-Bestandteil des ersten Rezeptors stammt oder einen Rezeptor verwendet, der von der gleichen Tierspezies wie der zweite Rezeptor stammt, wobei dann der erste Rezeptor entweder von einer anderen Tierspezies stammt oder/und mit einem Hapten oder Antigen markiert ist.

Als Rezeptoren werden im Rahmen der Erfindung entweder spezifisch bindefähige komplette Antikörper (polyklonal oder monoklonal), deren Antikörperfragmente oder Konjugate von Antikörpern oder Antikörperfragmenten mit Haptenen oder Antigenen verwendet. Vorzugsweise wird als erster Rezeptor ein kompletter Antikörper oder ein mit einem Hapten oder Antigen verbundener bzw. markierter Antikörper oder Antikörperfragment verwendet, besonders bevorzugt jeweils ein monoklonaler Antikörper.

Als zweiter Rezeptor wird ein Antikörper verwendet, welcher nur mit einem Teil des ersten Rezeptors bindefähig ist, besonders bevorzugt ein Antikörper, der nur mit dem Fc-Teil des ersten Rezeptors bindefähig ist. Alternativ läßt sich als zweiter Rezeptor auch ein Antikörper verwenden, der nur mit dem Haptenteil oder Antigenteil des ersten Rezeptors spezifisch bindefähig ist. Der zweite Rezeptor kann auch gegen den Fc-Teil von Immunoglobulin generell gerichtet sein.

Der dritte Rezeptor, der mit dem zweiten Rezeptor nicht kreuzreagieren darf, kann ein markierter, mit dem Antigen bindefähiger Antikörper sein, der aus einer anderen Tierspezies als der erste Rezeptor gewonnen wird.

Bevorzugt verwendet man jedoch einen dritten Rezeptor, welcher ein markiertes Fab-Fragment ist. Unter Fab-Fragment werden dabei im Rahmen der Erfindung auch (Fab)$_2$ und Fab'-Fragmente verstanden.

In diesem Fall kann Rezeptor 3 aus der gleichen Tierspezies wie der Antikörperbestandteil des ersten Rezeptors gewonnen werden. Dies gilt auch dann, wenn der erste Rezeptor aus einem Antikörper besteht, der ein Hapten oder Antigen trägt und der zweite Rezeptor nur mit diesem Hapten oder Antigen bindefähig ist. Besonders bevorzugt ist der dritte Rezeptor ein Teil, insbesondere ein Fab-Fragment, des ersten Rezeptors. Alternativ kann als dritter Rezeptor auch ein Antikörper verwendet werden, der von der gleichen Tierspezies wie der zweite Rezeptor stammt. Beide Alternativen haben den Vorteil, daß nur zwei Tierspezies für die drei Rezeptoren benötigt werden. Wenn der zweite Rezeptor nur mit dem Hapten oder Antigen des ersten Rezeptors bindefähig ist, wird sogar nur eine Tierspezies für alle drei Rezeptoren benötigt. Besonders bevorzugt enthalten der dritte und der erste Rezeptor monoklonale Antikörper bzw. Fragmente davon, die gegen verschiedene oder/und repetitive Determinanten des Antigens gerichtet sind.

Der dritte Rezeptor wird vorzugsweise in einer Menge eingesetzt, die größer ist als die Menge des nachzuweisenden Antigens. In diesem Falle, also bei Überschuß, ist keine exakte Dosierung bzw. genau bekannte Menge erforderlich. Der dritte Rezeptor ist in der dem Fachmann bekannten Weise markiert. Bevorzugt erfolgt die Markierung durch Kupplung mit einem Enzym, einer fluoreszierenden, chemilumineszierenden oder radioaktiven Substanz. Verfahren zur Markierung von derartigen Rezeptoren sind dem Fachmann bekannt, z. B. aus Clin. Chim. Acta 81 (1977) 1-40, und bedürfen hier keiner weiteren Erläuterung.

Der 1. und der 3. Rezeptor werden gelöst auf ein geeignetes Trägermaterial gebracht, z. B. auf saugfähiges Papier und durch Trocknung oder Lyophilisierung in eine leicht daraus ablösbare Form überführt.

Die Bindung des in fester Phase vorliegenden zweiten Rezeptors an ein unlösliches Trägermaterial kann nach den üblichen, dem Fachmann bekannten Methoden zur Fixierung biologisch aktiver Proteine an feste Trägersubstanzen vorgenommen werden. Sowohl eine kovalente als auch eine adsorptive Bindung ist geeignet. Bevorzugt wird jedoch, wegen der hierbei erzielbaren höheren Ausbeute und der vereinfachten Arbeitsweise, eine lediglich adsorptive Bindung, beispielsweise an Kunststoff oder ein Vlies wie z. B. Papier. Als geeignet erwiesen sich z. B. Reagenzgläschen aus Polystyrol und ähnlichen Kunststoffen, die adsorptiv an der Innenoberfläche mit dem zweiten Rezeptor beschichtet sind. Die Inkubation wird dann in diesem Röhrchen oder anders geformten Behälter durchgeführt und zur Phasentrennung genügt es, die flüssige Phase aus dem Röhrchen zu entfernen, beispielsweise durch Absaugen, Ausschütten und dergleichen. Nach Waschen des Röhrchens kann dann unmittelbar auch die Messung der Markierung darin vorgenommen werden, insbesondere wenn die Markierung mit einem Enzym erfolgt ist. Es genügt dann, einfach die Aktivität des Markierungsenzyms, beispielsweise Peroxidase oder $\beta$-Galactosidase, in der hierfür allgemein bekannten Weise zu messen, indem man ein bekanntes Nachweisreagenz für dieses Markierungsenzym, z. B. ein Farbreagenz, zusetzt und entweder in Anwesenheit der festen Phase oder nach Trennung mißt. Die gemessene Enzymaktivität ist dann ein Maß für die Menge an zu bestimmenden polyfunktionellen Antigen. Als Festphasenträger für den zweiten Rezeptor kommen aber auch teilchenförmige Substanzen, z. B. Ionenaustauscher, Molekularsiebmaterialien, Glaskörperchen, Kunststoffschläuche und dergleichen in Betracht. Besonders bevorzugt wird als Träger des zweiten Rezeptors ein poröser, schichtförmiger Träger wie Papier.

Falls die Markierung nicht mit einem Enzym sondern mit einer radioaktiven Substanz, einem Isotop, einer fluoreszierenden oder chemilumineszierenden Substanz erfolgt, so wird auch hier die Bestimmung nach einer der dem Fachmann wohl bekannten Methoden vorgenommen. Eine Beschreibung dieser Meßmethoden erübrigt sich daher hier.

Zur Durchführung des Verfahrens wird die Probelösung, die gemäß einem besonders vorteilhaften Merkmal der Erfindung sogar in unverdünnter Form z. B. als Blut, Plasma oder Serum, eingesetzt werden kann, mit dem ersten Trägermaterial ausreichend lange für die Ablösung der beiden Rezeptoren zusammengegeben, dann abgetrennt und mit dem zweiten Trägermaterial inkubiert. Die Kontaktzeit mit dem ersten Trägermaterial liegt im allgemeinen zwischen etwa 10 Sekunden und 10 Minuten. Vorteilhaft wird das Verfahren hierzu auf einer Vorrichtung, wie sie in der DE-A 34 25 008 beschrieben ist, durchgeführt.

Überraschenderweise wurde gefunden, daß erfindungsgemäß eine deutlich höhere Empfindlichkeit als bei der üblichen 2-Schritt-Sandwich-Methode und der 1-Schritt-Sandwich-Methode erreicht wird.

Wird gemäß der bevorzugten Ausführungsform der Erfindung ein gegen den Fc-Teil des ersten Rezeptors oder gegen das Hapten oder Antigen, welches mit dem ersten Rezeptor gekuppelt ist, gerichteter zweiter Rezeptor verwendet, so läßt sich hierbei eine weitere Steigerung der Empfindlichkeit erzielen, da eine eventuelle sterische Behinderung der Bindereaktion des Antigens mit der Bindungsstelle des ersten Rezeptors vermieden wird. Dies ist ein besonderer Vorteil, wenn Antigene mit besonders geringer Konzentration bestimmt werden müssen, wie dies z. B. bei der Bestimmung von Thyreotropin im Serum der Fall ist.

Ein weiterer Gegenstand der Erfindung ist ein Trockenreagenz zur Bestimmung eines polyvalenten Antigens gemäß dem Verfahren der Erfindung, gekennzeichnet durch

a) ein erstes mit zwei verschiedenen löslichen, mit dem Antigen bindefähige Rezeptoren 1 und 3, von denen Rezeptor 3 eine Markierung trägt, imprägniertes oder lyophilisiertes festes Trägermaterial und

b) ein zweites festes Trägermaterial, welches einen nur mit einem Teil des nichtmarkierten löslichen Rezeptors 1 bindefähigen unlöslichen Rezeptor 2, das von dem ersten Trägermaterial physikalisch getrennt vorliegt, enthält

mit der Maßgabe, daß der markierte dritte Rezeptor ein Fab-Antikörperfragment ist.

Die physikalische Trennung kann z. B. erfolgen, indem der unlösliche zweite Rezeptor und die löslichen ersten und dritten Rezeptoren auf getrennte Trägermaterialien aufgebracht werden. Dies kann dadurch erreicht werden, daß Rezeptor 1 und Rezeptor 3 gleichzeitig oder nacheinander in oder auf einem geeigneten Träger (beispielsweise Kunststoffröhrchen oder saugfähigen Träger wie Papier oder ähnliches) lyophilisiert oder imprägniert und der Rezeptor 2 adsorptiv oder kovalent an einen geeigneten Träger (beispielsweise Kunststoffröhrchen, Papier oder Partikel) gebunden werden. Die Probe mit dem zu bestimmenden polyvalenten Antigen wird zunächst mit dem Träger 1, der die löslichen ersten und dritten Rezeptoren enthält, und danach mit dem Träger 2, an dem der unlösliche Rezeptor 2 fixiert ist, in Kontakt gebracht. Alternativ können der erste und dritte Rezeptor einerseits und der zweite Rezeptor andererseits auf miteinander verbundenen Trägern vorliegen, die z. B. aus einem Papierstreifen bestehen, der die Rezeptoren auf verschiedenen, voneinander getrennten Stellen enthält.

Gemäß einer ersten bevorzugten Ausführungsform ist ein erfindungsgemäßes Reagenz dadurch gekennzeichnet, daß es einen ersten Rezeptor, der ein Antikörper ist und einen dritten Rezeptor, der ein enzymmarkiertes Fab-Fragment eines Antikörpers ist, enthält. Vorzugsweise enthält es auch Puffer und ein System zur Bestimmung der Enzymmarkierung.

Gemäß einer weiteren bevorzugten Ausführungsform des erfindungsgemäßen Reagenz zeichnet sich dieses dadurch aus, daß die Rezeptoren 1 und 3 monoklonal sind und gegen verschiedene Determinanten des Antigens gerichtet sind und aus der gleichen Tierspezies stammen.

Gemäß einer weiteren bevorzugten Ausführungsform des erfindungsgemäßen Reagenz zeichnet sich dieses dadurch aus, daß der zweite Rezeptor ein Antikörper gegen den Fc-Teil des ersten Rezeptors ist und der dritte Rezeptor aus der gleichen Tierspezies wie der zweite Rezeptor, der erste Rezeptor hingegen aus einer anderen Tierspezies stammt.

Gemäß einer weiteren bevorzugten Ausführungsform des erfindungsgemäßen Reagenz zeichnet sich dieses dadurch aus, daß alle drei Rezeptoren Antikörper aus derselben Tierspezies enthalten, der erste Rezeptor ein Hapten markierter monoklonaler Antikörper und der zweite Rezeptor gegen das Hapten des ersten Rezeptors gerichtet ist.

Die Rezeptoren 1 und 3 des erfindungsgemäßen Reagenz liegen auf einem festen Trägermaterial imprägniert oder lyophilisiert vor.

Die Erfindung eignet sich zur Bestimmung aller Antigene mit wenigstens zwei Antigendeterminanten. Beispiele hierfür sind Thyreotropin (TSH), alpha-1-Fetoprotein (AFP), hCG, carcinoembryonales Antigen, luteinisierendes Hormon (LH), follikelstimulierendes Hormon (FSH), $\beta_2$-Microglobulin, saure Prostataphosphatase, Prolactin, Ferritin und Insulin.

Bei der Erfindung kann Rezeptor 1 homogen mit dem Antigen reagieren. Diese Reaktion besitzt infolgedessen eine höhere Geschwindigkeitskonstante als bei Verwendung eines festphasengebundenen unlöslichen ersten Rezeptors. Da die Antikörperverluste bei der Fixierung (Unlöslichmachung) an die feste Phase vermieden werden, kann die Menge an erstem Rezeptor weiter verringert werden.

Es ist auch im Gegensatz zu dem in Clin. Chem. 27/6 (1981) beschriebenen Verfahren nicht erforderlich, den ersten und den dritten Rezeptor aus zwei unterschiedlichen Tierspezies zu gewinnen, die noch dazu so gewählt werden müssen, daß Rezeptor 2 nur mit Rezeptor 1 reagiert, nicht aber mit Rezeptor 3.

Die Gewinnung des Antikörpers für den Rezeptor 1 und für den Rezeptor 3 aus der gleichen Tierspezies ermöglicht es schließlich auch, zur Antiserumgewinnung Großtiere heranzuziehen.

Erfindungsgemäß wird schließlich auch die Empfindlichkeit gesteigert. So wurde für TSH eine Empfindlichkeit von weniger als 40 pg/ml (1 $\mu$U ≙ 170 pg/ml gefunden, während sie beim 2 site Assay (Sandwichverfahren) über 100 pg/ml liegt.

Die folgenden Beispiele erläutern die Erfindung weiter in Verbindung mit der Zeichnung. In dieser stellen dar:

Fig. 1    eine Eichkurve gemäß Beispiel 2,

Fig. 2    eine Eichkurve gemäß Beispiel 3,

Fig. 3    eine Eichkurve gemäß Beispiel 4.

Fig. 4    ein Einsatzelement für einen Zentrifugalanalysenautomaten, welches gemäß Beispiel 2 verwendet wird.

**Beispiel 1**

Bestimmung von Thyreotropin (TSH)

Rezeptor 1 und 3 stammen von derselben Tierspezies (Maus); Rezeptor 3 ist ein markiertes Fab-Fragment. Rezeptor 1 ist ein monoklonaler Antikörper, das Fab-Fragment in Rezeptor 3 stammt ebenfalls aus einem monoklonalen Antikörper, der gegen eine andere Determinante des TSH gerichtet ist als der monoklonale Antikörper von Rezeptor 1. Rezeptor 2 stammt aus Schaf und ist gegen den Fc-Teil der monoklonalen Mausantikörper gerichtet.

Die Entwicklung von monoklonalen Antikörper erfolgt nach der Methode von Köhler und Milstein Eur. J. Immunol. 6, 292 (1976).

a) Erfindungsgemäße Testführung

Reagenzien:

1. Inkubationspuffer (IP):

15 mM Natrium-Phosphat-Puffer, pH 7,4, 154 mM NaCl, 5 mM EDTA, 0,2 % RSA, pH 7,4.

2. Rezeptor 1:

Maus-anti-TSH-Antiserum (Rezeptor 1):

Die monoklonale Anti-TSH-Antikörper enthaltende Ascitesflüssigkeit aus Mäusen wird ad 1,8 M mit $NH_4SO_4$ versetzt. Das Präzipitat wird in einem Puffer aus 15 mM Natrium-Phosphat, pH 7,0 und 50 mM NaCl aufgenommen, und die so erhaltene Lösung wird einer Passage über DEAE-Cellulose unterworfen.

3. Rezeptor 3:

Anti-TSH-Antikörper (monoklonal), der eine andere antigene Determinante als Rezeptor 1 erkennt,

- Peroxidase-Konjugat (Rezeptor 3): Maus anti-TSH-Antiserum wird wie Rezeptor 1 aufgereinigt. Die anschließende Gewinnung von $(Fab)_2$ aus dem kompletten Antikörpermolekül erfolgt nach der im Beispiel 2 angegebenen Methode. Die Kopplung mit Meerrettich-Peroxidase erfolgt nach der Methode von Nakane (M.B. Wilson, P.K. Nakane "Recent Developments in the Periodate Method of Conjugating Horseradish Peroxidase to Antibodies", 1978, Elsevier, North Holland Biomedical Press p. 215-224 in "Immunofluorescence and Related Staining Techniques").

4. Rezeptor 2:

Adsorbermaterial mit fixiertem Schaf-anti-Maus-Fcγ-Antikörper (Rezeptor 2 Adsorber):

Schaf-anti-Maus-Fcγ-Antiserum wird ad 1,8 M mit $NH_4SO_4$ versetzt. Das Präzipitat wird in einem Puffer aus 15 mM Natriumphosphat, pH 7,0 und 50 mM NaCl aufgenommen und die so erhaltene Lösung einer Passage über DEAE-Cellulose unterworfen. Die IgG enthaltende Fraktion (Rezeptor 2) wird mit dem "Affinitätsadsorbens, Glutardialdehyd aktiviert" von Boehringer Mannheim (Best. Nr. 665 525) nach der Arbeitsvorschrift des Herstellers gekuppelt.

5. Indikatorreagenz:

1,8 mM ABTS[R] (2,2'-Azino-di[3-ethylbenzthiazolinsulfonat(6)]), 3,3 mM Natrium-Perborat in 100 mM Phosphat-Citrat-Puffer, pH 4,4.

Durchführung:

50 ng Rezeptor 1 und 170 mU Rezeptor 3 werden in Inkubationspuffer gelöst zusammen auf ein Vlies, bestehend aus kommerziellem Polyesterpapier von der Größe 6x6 mm, Dicke 1,0 mm, aufgetropft und bei Raumtemperatur getrocknet. Dieses Reagenz enthaltende Papiervlies wird Reagenzträger 1 genannt. Auf Reagenzträger 1 werden 40 μl der zu bestimmenden Probe bzw. bekannten Mengen Antigen enthaltenden Standardlösung pipettiert, anschließend wird das Vlies in einer Eppendorf-Zentrifuge sofort abzentrifugiert, wobei Reagenzträger 1 auf einem Eppendorf-Hütchen placiert wird und die eluierte Flüssigkeit im Hütchen während der Zentrifugation auf den Rezeptor 2, welcher auf Adsorber gekoppelt ist, trifft und mit diesem reagiert. Von Rezeptor 2 werden 2,5 mg Adsorbermaterial pro Test eingesetzt.

Auf einer Schüttelapparatur wird das Reaktionsgemisch 15 Minuten bei 37° C inkubiert.

Nach genau 15 Minuten werden die Reaktionsgefäße von der Apparatur herabgenommen und zentrifugiert.

Ein Aliquot des Überstandes wird mit einer Pipette entnommen und der gebildete Farbstoff über seine Extinktion bei 405 nm bestimmt.

Mit 2 verschiedenen TSH-Proben wurden folgende Extinktionswerte ermittelt:

EP 0 147 848 B1

| TSH (µU/ml) | $E_{405}$ nm/cm* |
|---|---|
| 0 | 0,74 |
| 50 | 7,54 |

\* Die Extinktionswerte wurden bei 0,2 cm Schichtdicke ermittelt und auf 1 cm Schichtdicke umgerechnet.

b) Einfacher Sandwich (Vergleich): Die Durchführung erfolgt mit den gleichen Reagenzien und in gleicher Weise wie unter a) beschrieben, aber mit folgenden Abänderungen:

1. 2,5 mg Adsorber-Rezeptor 2 wird 1 Stunde mit 20 µg Rezeptor 1 in 0,2 ml unter Schütteln vorinkubiert. Anschließend wird der Überstand abgesaugt und der Rückstand dreimal mit je 1 ml IP gewaschen.

2. Derart mit Rezeptor 1 vorbehandelter Adsorber-Rezeptor 2 wird 4 Stunden mit Probe und Rezeptor 3 auf einer Schüttelapparatur bei 37° C inkubiert, die Zugabe von löslichem Rezeptor 1 entfällt in dieser Variante.

Anschließend wird wie üblich der Überstand abgesaugt, der Rückstand gewaschen und die fixierte Enzymaktivität mit Indikatorreagenz nachgewiesen. Mit 2 verschiedenen TSH-Proben wurden folgende Extinktionswerte ermittelt:

| TSH (µU/ml) | $E_{405}$ nm/cm* |
|---|---|
| 0 | 0,20 |
| 50 | 2,87 |

\* Die Extinktionswerte wurden bei 0,2 cm Schichtdicke ermittelt und auf 1 cm Schichtdicke umgerechnet.

Die Empfindlichkeit ist deutlich geringer als nach a), obwohl bei b) wesentlich mehr Rezeptor 1 eingesetzt wird als bei a) (Verhältnis 1:400).

**Beispiel 2**

Bestimmung von Human-Choriogonadotropin (HCG)

Reagenzien:
Puffer A:
100 mmol Natriumphosphat, pH 7,3 (37° C)
2 mmol Magnesiumchlorid
0,9 % NaCl
0,5 % RSA (Rinderserumalbumin)
120 µg/ml Anti-HCG monoklonale Antikörper aus Maus (als Ascites) (Rezeptor 1)
100 mU/ml Anti-HCG-Antikörper-(Schaf)-Fab-Fragment-$\beta$-Galactosidase-Konjugat.
(Rezeptor 3)
Die Herstellung des Fab-Fragments erfolgt nach T. Kitagawa in "Enzyme Immunoassay E. Ishikawa, T.

8

EP 0 147 848 B1

Kawai, K. Miyai eds Igaku-Shoin, Tokio/New York 1981, S.81-89". Die kovalente Kopplung von $\beta$-Galactosidase an Antikörper bzw. AK-Fragmente erfolgt nach der Methode: R.R. Porter, Biochem. J. 73 (1959), 119.
Substrat-Lösung:
wie Puffer A, zusätzlich 5 mmol (ONPG) ortho-Nitrophenylgalactosid.

1. Die Herstellung von Reagenzträger 1 erfolgt analog zu Beispiel 1 in der Weise, daß von Lösung A 40 $\mu$l auf ein Vlies, bestehend aus kommerziellem Polyesterpapier aufgetropft und anschließend bei Raumtemperatur aufgetrocknet werden. Die Lagerung der Vliese bis zu ihrer Verwendung erfolgt bei 4°C und relativer Luftfeuchte $\leq$ 20%.

2. Herstellung von Reagenzträger 2: Auf ein Cellulosevlies werden nach dem Bromcyan-Aktivierungsverfahren (DE-OS 1768512) (M-Fc$\gamma$)-Antikörper vom Schaf (IgG-Fraktion) fixiert, wobei pro Gramm Fasermaterial 10 mg Antikörper zur Fixierung angeboten werden. Durch Waschen wird ungekoppelter Antikörper entfernt und das Vlies schonend bei Raumtemperatur getrocknet. Die Lagerung der Vliese erfolgt analog Reagenzträger 1.

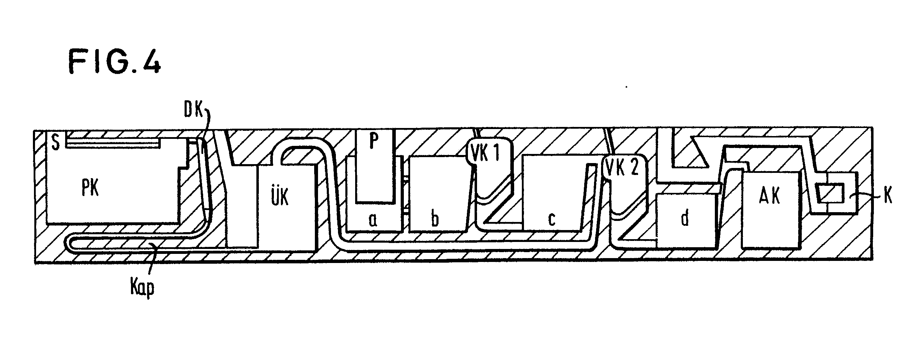

Die Bestimmung von HCG mit Hilfe dieser beiden Reagenzträger 1 und 2 erfolgt unter Zuhilfenahme der in der deutschen Patentanmeldung 24 25 008.5 beschriebenen Vorrichtung zur Durchführung analytischer Bestimmungen. Diese offenbart ein Rotoreinsatzelement für Zentrifugalanalysenautomaten, bestehend aus einem Formkörper, der eine Probenauftragskammer, die mit einer Mehrzahl von Reagenzfeldern in Verbindung steht, die jeweils ein mit einem bestimmten Reagenz imprägniertes saugfähiges Trägermaterial enthalten, wenigstens eine Mischventilkammer und eine Meßkammer aufweist, die zusammen einen Probenflüssigkeitstransportweg bilden, der von radial innen nach radial weiter außen führt, wenn das Einsatzelement auf dem Rotor befestigt ist und weiter wenigstens eine weitere Kammer für die Aufnahme einer Flüssigkeit und einen Transportweg, der von dieser Kammer zur Meßführung führt und mit dem Probenflüssigkeitstransportweg mindestens teilweise identisch ist, aufweist. Dabei führt der Probenflüssigkeitstransportweg von einer Probenauftragskammer (P) über eine mit saugfähigem Material gefüllte, Puffer enthaltende Kammer (a), eine Kammer (c) und eine zwischen den Kammern (a) und (c) angeordnete erste Ventilkammer (VK1) zu einer zweiten Ventilkammer (VK2) und von dieser über die Kammer (d) und über eine Auffangkammer (AK) zur Meßkammer (K). Zur Aufnahme einer weiteren Flüssigkeit ist eine als Pumpenkammer ausgebildete Substratkammer (PK) vorgesehen, welche über eine aus Dosierkammer (DK) und Kapillare (Kap) bestehende Dosiereinrichtung und eine Überlaufkammer (ÜK) mit der zweiten Ventilkammer (VK2) verbunden ist. Fig. 4 zeigt schematisch das verwendete Rotoreinsatzelement. Hierbei wird Reagenzträger 1 auf Feld c des Einsatzelementes placiert und Reagenzträger 2 auf Feld d. Die Reaktionsführung entspricht im wesentlichen der von Beispiel 1 dieser Patentanmeldung. Dabei werden 40 $\mu$l Probe durch eine Öffnung am oberen Rand direkt auf das Feld a pipettiert. Die Probe ist unverdünnt.

Durch ein geeignetes Programm, wo hohe Drehzahlen mit Stillstand abwechseln, werden dann Probe und Substrat in Richtung Trennmatrix und Küvette gefördert. Im folgenden steht Zentrifugation für hohe Drehzahl, zwischengeschaltete Schritte mit niedriger Drehzahl dienen der sensibleren Steuerung des Flüssigkeitstransportes. Die Substrat/Waschlösung wird durch die Dosierkapillare (DK) in gleich große Schlucke (Portionen) geteilt.

| | |
|---|---|
| 1. Zentrifugation | Probe und Probepuffer werden in VK 1 geschleudert, der erste Schluck ist in der Dosierkammer DK. |
| 1. Stillstand | Probe läuft auf Feld c und löst die Rezeptoren 1 und 3. Der 1. Schluck Substratlösung geht in die Überlaufkammer ÜK. |
| 2. Zentrifugation | HCG und Rezeptoren 1 und 3 gelangen in die VK 2, es wird zentrifugiert, um homogene Mischung der Reagenzien zu erreichen. Der 1. Schluck Substratlösung wird in ÜK zurückgehalten, der 2. Schluck Substratlösung geht in die Dosierkammer DK. |
| 2. Stillstand | Probeseitig wird die Flüssigkeit auf Feld d transportiert, es folgt ein Stillstand von 5 Minuten, während der Zeit sich der Komplex an den Träger bindet. Es bindet auch nicht komplexiertes Anti HCG. Am Ende der Reaktion befindet sich noch nicht komplexiertes Fab-Honjugat ungebunden in der Lösung. Der 2. Schluck Substratlösung geht in die ÜK. |
| 3. Zentrifugation | Die aus dem Probekanal gekommene Flüssigkeit wird in die Auffangkammer (AK) zentrifugiert, mit ihr der Überschuß an Fab-Konjugat. Der 2. Substratschluck wird in ÜK gehalten. |
| | Der 3. Substratschluck befindet sich in Dosierkammer DK. |
| 3. Stillstand | Der 3. Substratschluck wird nach ÜK befördert. |
| 4. Zentrifugation | Schluck 4 nach DK |
| | Schluck 3 nach VK 2 |

9

| 4. Stillstand | Schluck 4 nach ÜK |
| | Schluck 3 nach Feld d |
| | Der 1. Waschschluck befindet sich auf Reagenzträger 2. |
| 5. Zentrifugation | Schluck 5 nach DK |
| | Schluck 4 nach VK2 |
| | Schluck 3 nach AK |
| 5. Stillstand | Schluck 5 nach ÜK |
| | Schluck 4 nach d |
| | 2. Waschschluck auf Reagenzträger 2 |
| 6. Zentrifugation | Schluck 6 nach DK |
| | Schluck 5 nach VK2 |
| | Schluck 4 nach AK |
| 6. Stillstand | Schluck 6 nach ÜK |
| | Schluck 5 nach d |
| | 3. Waschschluck auf Reagenzträger 2 |
| 7. Zentrifugation | Schluck 7 nach DK |
| | Schluck 6 nach VK2 |
| | Schluck 5 nach AK |
| 7. Stillstand | Schluck 7 nach ÜK |
| | Schluck 6 nach d |
| | 4. Waschschluck auf Reagenzträger 2 |
| 8. Zentrifugation | Schluck 8 nach DK |
| | Schluck 7 nach VK2 |
| | Schluck 6 nach AK |
| 8. Stillstand | Schluck 8 nach ÜK |
| | Schluck 7 nach d |
| | Nachweisschluck auf Reagenzträger 2. In 5 Minuten Reaktion wird das Substrat von dem auf Reagenzträger 2 gebundenen Enzym, d. h. einer Enzymmenge, die durch die Komplexbildung proportional zur eingesetzten Menge HCG ist, gespalten und es bildet sich die zu messende Farbe. |
| 9. Zentrifugation | Die vom Reagenzträger 2 kommende Flüssigkeit füllt mit einem ersten Aliquot die AK vollständig und der Rest wird in die Küvette gefördert. In der Küvette erfolgt die Messung der gebildeten Farbe bei 578 nm. |

Der Transfer der Probe von Feld c nach d erfolgt innerhalb sehr kurzer Zeit ($\leq$ 60 Sekunden). Das letztendlich zum Nachweis führende Substratvolumen beträgt ebenfalls 40 $\mu$l, so daß keinerlei Probenverdünnung bis zur Signalmessung erfolgt.

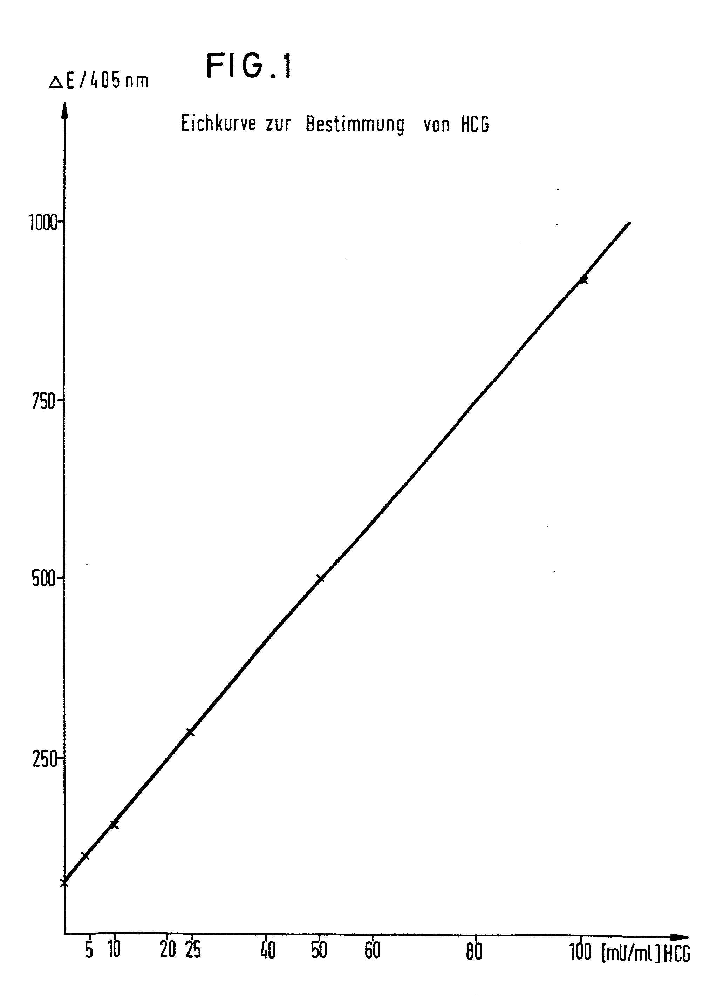

Unter Verwendung von Eichserum wird die Eichkurve von Fig. 1 erhalten, die den Bereich 0 bis 100 mU HCG/ml (standardisiert nach dem 1. IRP-Standard für HCG) erfaßt und eine ausreichend empfindliche Messung von HCG in Serum und Plasma ermöglicht.

**Beispiel 3**

Bestimmung von alpha-1-Fetoprotein (AFP)

Verwendung eines haptenmarkierten 1. Rezeptors, wobei sämtliche 3 Antikörper aus derselben Tierspezies stammen.

Die Durchführung bzw. die verwendeten Puffer entsprechen denen im Beispiel 2.

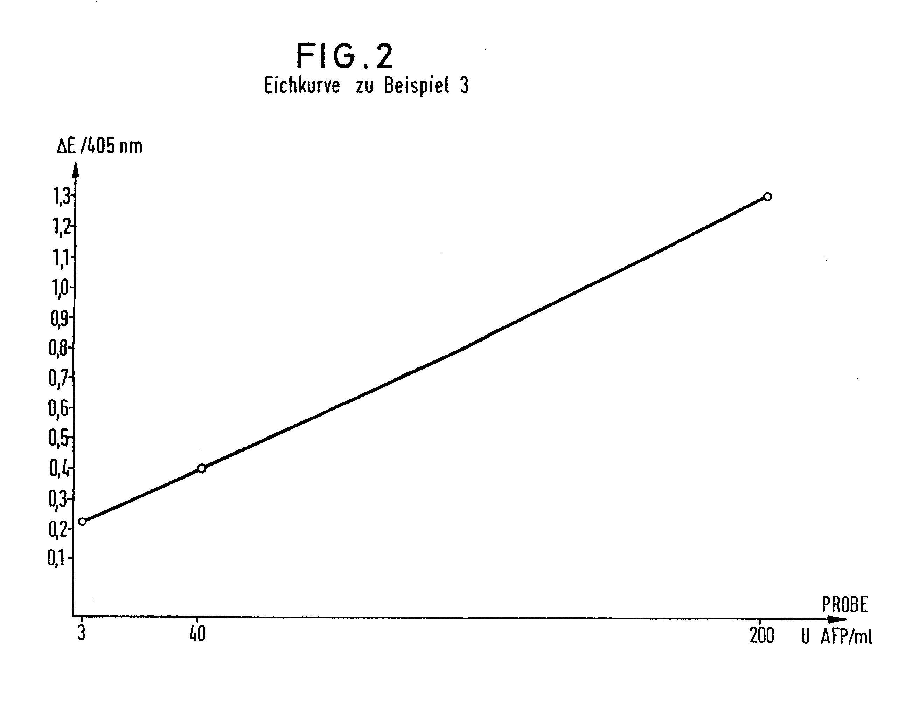

Als Rezeptor 1 werden Anti-AFP-Antikörper vom Schaf (100 ng/ml), die mit Digoxin markiert sind (nach DE-Patentschrift 25 37 129) als Rezeptor 3 werden Anti-AFP-Antikörper vom Schaf, markiert mit $\beta$-Galactosidase gemäß der bei HCG (Beispiel 2) genannten Methode, eingesetzt und als festphasengekoppelter Rezeptor 2 werden Papierscheibchen aus Polyester-Papier verwendet, an die adsorptiv Antikörper gegen Digoxin vom Schaf gekoppelt werden. Das Beschichtungsverfahren entspricht den von Plastikröhrchen bekannten Verfahren. Die erhaltene Eichkurve zeigt Fig. 2 der Zeichnung.

**Beispiel 4**

Bestimmung von Thyreotropin (TSH)

Reagenzien:

1. Inkubationspuffer (IP): 15 mM Natrium-Phosphat-Puffer, pH 7,4, 154 mM NaCl, 5 mM EDTA, 0,2 % RSA, pH 7,4.

2. Rezeptor 1: Schaf-anti-TSH-Antiserum: Das Rohserum wird ad 1,8 M mit $NH_4SO_4$ versetzt. Das Präzipitat wird in einem Puffer aus 15 mM Natrium-Phosphat, pH 7,0 und 50 mM NaCl aufgenommen, und die so erhaltene Lösung wird einer Passage über DEAE-Cellulose unterworfen. Die IgG enthaltende Fraktion wird anschließend von mit der alpha-Kette von TSH reagierenden Anteilen durch Passage über einen mit HCG beladenen Immunadsorber befreit. Das Eluat wird auf einen mit TSH beladenen Immunadsorber gegeben. Nach Waschen des Adsorbers erfolgt die Elution der gegen TSH immunreaktiven Antikörper mit 1 M Propionsäure. Das Eluat wird anschließend gegen IP dialysiert, gegebenenfalls durch Ultrafiltration eingeengt.

3. Rezeptor 3: Antikörper-Peroxidase- Konjugat: Ein weiteres Schaf-anti-TSH-Antiserum wird wie Rezeptor 1 aufgereinigt und anschließend daraus $(Fab)_2$-Fragmente gewonnen. Die Herstellung der $(Fab)_2$-Fragmente erfolgt nach E. Lamoye et al. J. Immunol. Methods 56, 235-243 (1983). Die Kopplung mit Meerrettich-Peroxidase erfolgt nach der Methode von Nakane (m.B. Wilson, P.K. Nakane "Recent Developments in the Periodat Method of Conjugating Horseradish Peroxidase to Antibodies", 1978, Elsevier, North Holland Biomedical Press, p. 215-224 in "Immunofluorescence and Related Staining Techniques").

4. Rezeptor 2: Adsorbermaterial mit fixiertem Esel-anti-Schaf-Fc$\gamma$-Antikörper: Esel-anti-Schaf-Fc$\gamma$-Antiserum wird ad 1,8 M mit $NH_4SO_4$ versetzt. Das Präzipitat wird in einem Puffer aus 15 mM Natriumphosphat, pH 7,0 und 50 mM NaCl aufgenommen, und die so erhaltene Lösung einer Passage über DEAE-Cellulose unterworfen. Die IgG enthaltende Fraktion (Rezeptor 2) wird mit dem "Affinitätsadsorbens, Glutardialdehyd-aktiviert" von Boehringer Mannheim (Best. Nr. 665 525) nach der Arbeitsvorschrift des Herstellers gekuppelt.

5. Indikatorreagenz: 1,8 mM ABTS$^R$ (2,2'-Azino-di-3-ethylbenzthiazolinsulfonat-(6), 3,3 mM Natrium-Perborat in 100 mM Phosphat-Citrat-Puffer, pH 4,4.

Durchführung:

Die Durchführung entspricht dem in Beispiel 1 dargestellten Verfahren, wobei in diesem Fall 100 ng Rezeptor 1 und 100 mU von Rezeptor 3, gelöst in Inkubationspuffer (40 µl) auf ein Cellulosevlies der Größe 6x6 mm aufgetropft werden. Nach Trocknung erfolgt die Lagerung der Vliese bis zu ihrer Verwendung wieder bei 4° C.

Die weitere Durchführung entspricht der in Beispiel 1 dargestellten Arbeitsweise. Als Probe werden 40 µl eingesetzt.

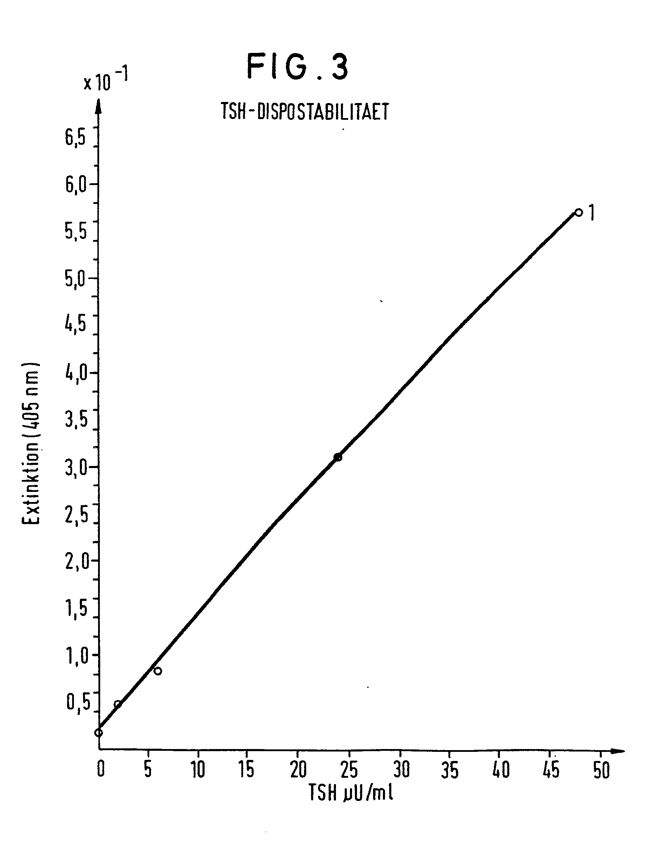

Eine so erhaltene Eichkurve zeicht Fig. 3.

## Patentansprüche

1. Verfahren zur Bestimmung eines polyvalenten Antigens durch Inkubation mit drei verschiedenen Rezeptoren, von denen der erste und der dritte in flüssiger Phase gelöst vorliegen und mit dem Antigen bindefähig sind, der zweite Rezeptor in fester Phase vorliegt und nur mit einem Teil des ersten Rezeptors bindefähig ist und der dritte Rezeptor eine Markierung trägt und mit dem ersten und zweiten Rezeptor nicht kreuzreagiert, Trennung der festen von der flüssigen Phase und Messung der Markierung in einer der Phasen, **dadurch gekennzeichnet,** daß man eine das Antigen enthaltende Lösung mit einem ersten festen Trägermaterial zusammenbringt, welches den ersten und den dritten Rezeptor in ablösbarer Form imprägniert oder lyophilisiert enthält und nach Ablösung der Rezeptoren die erhaltene Lösung sofort mit einem zweiten festen Trägermaterial kontaktiert, welches den zweiten Rezeptor in nicht ablösbarer Form enthält, danach die Flüssigkeit vom zweiten festen Trägermaterial abtrennt und die Markierung mißt, wobei man als dritten Rezeptor entweder ein Fab-Antikörperfragment verwendet, das aus der gleichen Tierspezies wie der Antikörper-Bestandteil des ersten Rezeptors stammt oder einen Rezeptor verwendet, der von der gleichen Tierspezies wie der zweite Rezeptor stammt, wobei dann der erste Rezeptor entweder von einer anderen Tierspezies stammt oder/und mit einem Hapten oder Antigen markiert ist.

2. Verfahren nach Anspruch 1, **dadurch gekennzeichnet,** daß man als ersten Rezeptor einen Antikörper verwendet.

3. Verfahren nach Anspruch 1, **dadurch gekennzeichnet,** daß man als ersten Rezeptor einen mit einem Hapten oder Antigen markierten Antikörper verwendet.

4. Verfahren nach Anspruch 1,
**dadurch gekennzeichnet,**
daß man als ersten Rezeptor ein Antikörperfragment verwendet, welches mit einem Hapten oder Antigen markiert ist.

5. Verfahren nach Anspruch 2, **dadurch gekennzeichnet,** daß man einen zweiten Rezeptor verwendet, der nur mit dem Fc-Teil des ersten Rezeptors bindefähig ist.

6. Verfahren nach Anspruch 3 oder 4,
**dadurch gekennzeichnet,**
daß man einen zweiten Rezeptor verwendet, der nur mit dem Haptenteil oder Antigenteil des ersten Rezeptors bindefähig ist.

7. Verfahren nach Anspruch 5, **dadurch gekennzeichnet,** daß man einen zweiten Rezeptor verwendet, der gegen den Fc-Teil von Immunoglobulin gerichtet ist.

8. Verfahren nach Anspruch 1,
**dadurch gekennzeichnet,**
daß man als dritten Rezeptor einen Teil des ersten Rezeptors verwendet.

9. Verfahren nach Anspruch 3 oder 4,
**dadurch gekennzeichnet,**
daß man einen dritten Rezeptor verwendet, der aus einem markierten kompletten Antikörper besteht.

10. Verfahren nach Anspruch 3 oder 4,
**dadurch gekennzeichnet,**
daß alle drei
Rezeptoren Antikörper bzw. Fragmente davon enthalten, die aus der gleichen Tierspezies stammen.

11. Verfahren nach einem der vorhergehenden Ansprüche,
**dadurch gekennzeichnet,**
daß man einen dritten Rezeptor verwendet, welcher mit einem Enzym, einer fluoreszierenden, chemilumineszierenden oder radioaktiven Substanz markiert ist.

12. Verfahren nach einem der vorhergehenden Ansprüche,
**dadurch gekennzeichnet,**
daß man unverdünnte Probelösung einsetzt.

13. Trockenreagenz zur Bestimmung eines polyvalenten Antigens nach einem Verfahren gemäß einem der vorhergehenden Ansprüche, **gekennzeichnet, durch**
a) ein erstes mit zwei verschiedenen löslichen, mit den Antigen bindefähigen Rezeptoren 1 und 3, von denen Rezeptor 3 eine Markierung trägt, imprägniertes oder lyophilisiertes festes Trägermaterial und
b) ein zweites festes Trägermaterial, welches einen nur mit einem Teil des nichtmarkierten löslichen Rezeptors 1 bindefähigen unlöslichen Rezeptor 2 enthält, das von dem ersten Trägermaterial physikalisch getrennt vorliegt,
mit der Maßgabe, daß der markierte dritte Rezeptor ein Fab-Antikörperfragment ist.

14. Reagenz nach Anspruch 13, **dadurch gekennzeichnet,** daß der erste Rezeptor ein Antikörper ist und der dritte Rezeptor ein enzymmarkiertes Fab-Fragment eines Antikörpers ist.

15. Reagenz nach Anspruch 14, **dadurch gekennzeichnet,** daß die Rezeptoren 1 und 3 monoklonal sind und gegen verschiedene und/oder repetitive Determinanten des Antigens gerichtet sind und aus der gleichen Tierspezies stammen.

16. Reagenz nach Anspruch 14, **dadurch gekennzeichnet,** daß der zweite Rezeptor ein Antikörper gegen den Fc-Teil des ersten Rezeptors ist und der dritte Rezeptor aus der gleichen Tierspezies wie der zweite Rezeptor, der erste Rezeptor hingegen aus einer anderen Tierspezies stammt.

EP 0 147 848 B1

17. Reagenz nach Anspruch 15, **dadurch gekennzeichnet,** daß alle drei Rezeptoren Antikörper aus derselben Tierspezies enthalten, der erste Rezeptor ein Hapten- bzw. Antigen markierter monoklonaler Antikörper und der zweite Rezeptor gegen das Hapten- bzw. Antigen des ersten Rezeptors gerichtet ist.

18. Reagenz nach Anspruch 14, **dadurch gekennzeichnet,** daß der erste Rezeptor ein kompletter Antikörper, der dritte Rezeptor aus der gleichen Tierspezies wie der erste Rezeptor und der zweite Rezeptor ein gegen den Fc-Teil des ersten Rezeptors gerichteter Antikörper ist.

19. Reagenz nach einem der Ansprüche 13 bis 18,
**dadurch gekennzeichnet,**
daß es Puffer und ein System zur Bestimmung der Enzymmarkierung enthält.

20. Reagenz nach einem der Ansprüche 13 bis 19,
**dadurch gekennzeic net,**
daß der dritte Rezeptor mit Peroxidase oder alpha- bzw. $\beta$-Galactosidase markiert ist.

**Claims**

1. Process for the determination of a polyvalent antigen by incubation with three different receptors, the first and third of which are present dissolved in liquid phase and are bindable with the antigen, the second receptor is present in solid phase and is only bindable with a part of the first receptor and the third receptor carries a labelling and does not cross-react with the first and second receptor, separation of the solid from the liquid phase and measurement of the labelling in one of the phases, characterised in that one brings a solution containing the antigen together with a first solid carrier material, which contains, impregnated or lyophilised, the first and the third receptor in removable form and, after removal of the receptors, immediately contacts the solution obtained with a second solid carrier material which contains the second receptor in non-removable form, thereafter separates the liquid from the second solid carrier material and measures the labelling, whereby, as third receptor, one uses either a Fab antibody fragment which originates from the same animal species as the antibody component of the first receptor or uses a receptor which originates from the same animal species as the second receptor, whereby the first receptor originates either from another animal species and/or is labelled with a hapten or antigen.

2. Process according to claim 1, characterised in that one uses an antibody as first receptor.

3. Process according to claim 1, characterised in that one uses an antibody labelled with a hapten or antigen as first receptor.

4. Process according to claim 1, characterised in that, as the first receptor, one uses an antibody fragment which is labelled with a hapten or antigen.

5. Process according to claim 2, characterised in that one uses a second receptor which is only bindable with the Fc part of the first receptor.

6. Process according to claim 3 or 4, characterised in that one uses a second receptor which is only bindable with the hapten part or the antigen part of the first receptor.

7. Process according to claim 5, characterised in that one uses a second receptor which is directed against the Fc part of immunoglobulin.

8. Process according to claim 1, characterised in that one uses a part of the first receptor as third receptor.

9. Process according to claim 3 or 4, characterised in that one uses a third receptor which consists of a labelled complete antibody.

10. Process according to claim 3 or 4, characterised in that all three receptors contain antibodies or fragments thereof which originate from the same animal species.

11. Process according to one of the preceding claims, characterised in that one uses a third receptor which is labelled with an enzyme, a fluorescing, chemiluminescing or radioactive substance.

12. Process according to one of the preceding claims, characterised in that one uses an undiluted sample solution.

13. Dry reagent for the determination of a polyvalent antigen according to a process according to one of the preceding claims, characterised by
   a) a first solid carrier material impregnated or lyophilised with two different soluble receptors 1 and 3, bindable with the antigen, of which receptor 3 carries a labelling, and
   b)m a second solid carrier material, which is present physically separated from the first carrier material, which contains an insoluble receptor 2 only bindable with a part of the non-labelled soluble receptor 1,
   with the proviso that the labelled third receptor is a Fab antibody fragment.

14. Reagent according to claim 13, characterised in that the first receptor is an antibody and the third receptor is an enzyme-labelled Fab fragment of an antibody.

15. Reagent according to claim 14, characterised in that the receptors 1 and 3 are monoclonal and are directed against different and/or repetitive determinants of the antigen and originate from the same animal species.

16. Reagent according to claim 14, characterised in that the second receptor is an antibody against the Fc part of the first receptor and the third receptor originates from the same animal species as the second receptor, whereas the first receptor originates from another animal species.

17. Reagent according to claim 15, characterised in that all three receptors contain antibodies from the same animal species, the first receptor is a hapten- or antigen-labelled monoclonal antibody and the second receptor is directed against the hapten or antigen first receptor.

18. Reagent according to claim 14, characterised in that the first receptor is a complete antibody, the third receptor is from the same animal species as the first receptor and the second receptor is an antibody directed against the Fc part of the first receptor.

19. Reagent according to one of claims 13 to 18, characterised in that it contains a buffer and a system for the determination of the enzyme labelling.

20. Reagent according to one of claims 13 to 19, characterised in that the third receptor is labelled with peroxidase or alpha- or $\beta$-galactosidase.

**Revendications**

1. Procédé pour déterminer un antigène polyvalent par incubation avec trois récepteurs différents, parmi lesquels le premier et le troisième sont présents à l'état dissous en phase liquide et sont capables de se lier à l'antigène, le deuxième récepteur est présent en phase solide et n'est capable de se lier qu'à une partie du premier récepteur, et le troisième récepteur porte un marquage et ne réagit pas de manière croisée avec le premier et le deuxième récepteur, séparation de la phase solide d'avec la phase liquide et mesure du marquage dans l'une des phases, caractérisé en ce que l'on met en contact une solution contenant l'antigène avec un premier matériau de support solide qui contient le premier et le troisième récepteur imprégnés ou lyophilisés sous forme séparable et, après séparation des récepteurs l'on met la solution obtenue immédiatement en contact avec un deuxième matériau de support solide qui contient le deuxième récepteur sous forme non séparable, puis l'on sépare le liquide du deuxième matériau de support solide et l'on mesure le marquage, en utilisant comme troisième récepteur un fragment Fab d'anticorps qui provient de la même espèce animale que le constituant d'anticorps du premier récepteur ou un récepteur qui provient de la même espèce animale que le deuxième récepteur, le premier récepteur provenant alors d'une autre espèce animale et/ou étant marqué avec un haptène ou un antigène.

2. Procédé selon la revendication 1, caractérisé en ce que l'on utilise un anticorps comme premier récepteur.

3. Procédé selon la revendication 1, caractérisé en ce que l'on utilise comme premier récepteur un anticorps marqué avec un haptène ou un antigène.

4. Procédé selon la revendication 1, caractérisé en ce que l'on utilise comme premier récepteur un fragment d'anticorps qui est marqué avec un haptène ou un antigène.

5. Procédé selon la revendication 2, caractérisé en ce que l'on utilise un deuxième récepteur qui n'est capable de se lier qu'avec la partie Fc du premier récepteur.

6. Procédé selon la revendication 3 ou 4, caractérisé en ce que l'on utilise un deuxième récepteur qui n'est capable de se lier qu'avec la partie haptène ou la partie antigène du premier récepteur.

7. Procédé selon la revendication 5, caractérisé en ce que l'on utilise un deuxième récepteur qui est dirigé contre la partie Fc de l'immunoglobuline.

8. Procédé' selon la revendication 1, caractérisé en ce que l'on utilise comme troisième récepteur une partie du premier récepteur.

9. Procédé selon la revendication 3 ou 4, caractérisé en ce que l'on utilise un troisième récepteur qui consiste en un anticorps complet marqué.

10. Procédé selon la revendication 3 ou 4, caractérisé en ce que les trois récepteurs contiennent tous des anticorps ou des fragments d'anticorps qui proviennent de la même espèce animale.

11. Procédé selon l'une des revendications précédentes, caractérisé en ce que l'on utilise un troisième récepteur qui est marqué avec un enzyme ou une substance fluorescente, chemiluminescente ou radioactive.

12. Procédé selon l'une des revendications précédentes, caractérisé en ce que l'on utilise une solution échantillon non diluée.

13. Réactif sec pour déterminer un antigène polyvalent selon un procédé selon l'une des revendications précédentes, caractérisé par
   a) un premier matériau de support solide imprégné ou lyophilisé avec deux récepteurs différents 1 et 3 solubles, capables de se lier avec l'antigène, parmi lesquels le récepteur 3 porte un marquage, et
   b) un deuxième matériau de support solide qui contient un récepteur 2 insoluble, capable de se lier seulement avec une partie du récepteur 1 soluble non marqué, qui est séparé physiquement du premier matériau de support,
   avec la condition que le troisième récepteur marqué soit un fragment Fab d'anticorps.

14. Réactif selon la revendication 13, caractérisé en ce que le premier récepteur est un anticorps et le troisième récepteur est un fragment Fab à marquage enzymatique d'un anticorps.

15. Réactif selon la revendication 14, caractérisé en ce que les récepteurs 1 et 3 sont monoclonaux et sont dirigés contre des déterminants différents et/ou répétitifs de l'antigène et proviennent de la même espèce animale.

16. Réactif selon la revendication 14, caractérisé en ce que le deuxième récepteur est un anticorps contre la partie Fc du premier récepteur et le troisième récepteur provient de la même espèce animale que le deuxième récepteur, tandis que le premier récepteur provient d'une autre espèce animale.

17. Réactif selon la revendication 15, caractérisé en ce que les trois récepteurs contiennent tous des anticorps provenant de la même espèce animale, le premier récepteur contenant un anticorps monoclonal marqué avec un haptène ou un antigène et le deuxième récepteur étant dirigé contre l'haptène ou l'antigène du premier récepteur.

**18.** Réactif selon la revendication 14, caractérisé en ce que le premier récepteur est un anticorps complet, le troisième récepteur provient de la même espèce animale que le premier récepteur et le deuxième récepteur est un anticorps dirigé contre la partie Fc du premier récepteur.

**19.** Réactif selon l'une des revendications 13 à 18, caractérisé en ce qu'il contient des tampons et un système pour déterminer le marquage enzymatique.

**20.** Réactif selon l'une des revendications 13 à 19, caractérisé en ce que le troisième récepteur est marqué avec une peroxydase ou l'alpha- ou $\beta$-galactosidase.

FIG.1

ΔE/405 nm

Eichkurve zur Bestimmung von HCG

# FIG.2

## Eichkurve zu Beispiel 3

ΔE /405 nm

1,3 1,2 1,1 1,0 0,9 0,8 0,7 0,6 0,5 0,4 0,3 0,2 0,1

3  40  200  U AFP/ml

PROBE

FIG.3

TSH-DISPOSTABILITAET

FIG.4